# EUROPEAN PATENT APPLICATION

(11) **EP 4 425 180 A1**
(43) Date of publication of application: **04.09.2024**
(21) Application number: 23188417.2
(22) Date of filing: 28.07.2023
(51) Int. Cl.: G01N 33/543, G01N 21/64, G01N 33/58

(54) **IMMUNOASSAY USING MAGNETIC MICROSPHERES**

(71) Applicant: MABTECH AB, 131 28 Nacka Strand (SE)
(72) Inventor: Smedman, Christian, SE-131 28 Nacka Strand (SE)
(74) Representative: EIP

(57) **Abstract**

A method (10) and system (100) for determining presence of a plurality of fluorophore-labelled detection molecules (114) are disclosed, wherein the detection molecules are bound to a plurality of particles (110) arranged on a planar surface (120). Accordingly, a sample comprising the particles suspended in a liquid medium (115) is provided (12) and the particles exposed (14) to an alternating magnetic field (B) to reduce a tendency of the particles to aggregate on the planar surface. The particles are allowed (16) to settle on the planar surface, whereby they are illuminated (18) and photons emitted from the illuminated detection molecules are detected (20). Presence of the detection molecules is then determined (22) based at least in part on the detected photons.

## Description

### Technical Field

The present invention relates to investigative techniques such as assays, and specifically to immunoassays using magnetic microspheres.

### Background

Immunoassays are widely used in medical diagnostics and research to detect and quantify specific analytes, such as proteins or other molecules in a sample, including hormones, antibodies, antigens, and other biomarkers of health or disease. Generally, immunoassays involve binding the analyte to an analyte-specific capture molecule to form a capture molecule-analyte complex. Unbound substances may then be separated from the complexes before the complexes are detected and quantified. The detection and quantification often involve a secondary reaction where a detectable signal is produced, typically via fluorescence emission from a detection molecule that is bound to the complex. The intensity of the signal is proportional to the concentration of the analyte in the sample.

In flow-based assays, the capture molecules may be attached to the surface of particles, commonly referred to as microspheres. Once the complexes are formed, the microspheres are passed through a flow cytometer. This instrument typically uses lasers to excite the fluorescent detection molecules and the dyed particle itself, allowing the emitted fluorescence to be measured for each passing microsphere. While flow-based assays may be powerful tools for biological analysis, they are associated with drawbacks. One disadvantage relates to complexity of the instrument which can be expensive to maintain and require skilled technicians and training to operate. Another disadvantage concerns sample preparation and handling, as the samples tend to clog the cytometer over time and will inherently contaminate the instrumentation with potentially dangerous pathogens.

Hence, there is a need for improved and more efficient techniques for detecting and quantifying analytes in a sample using microspheres.

### Summary

It is an object of the present invention to provide a method and a system which address at least some of the above-mentioned drawbacks. This object is achieved by the invention as defined in the independent claims.

According to a first aspect of the present invention, there is provided a method for determining presence of a plurality of fluorophore-labelled detection molecules bound to a plurality of particles arranged on a planar surface. The method comprises providing a sample comprising the particles suspended in a liquid medium, exposing the particles to an alternating magnetic field to reduce a tendency of the particles to aggregate on the planar surface, and allowing the particles to settle on the planar surface. The settled particles are illuminated, photons emitted from the illuminated detection molecules are detected, and the presence of the detection molecules is determined based at least in part on the detected photons.

According to a second aspect, a system for determining presence of a plurality of fluorophore-labelled detection molecules bound to a plurality of particles arranged on a planar surface is provided. The system comprises a magnetic field generator configured to expose a sample, comprising the particles suspended in a liquid medium, to an alternating magnetic field to reduce a tendency of the particles to aggregate on the planar surface. The system further comprises an illumination means for illuminating the particles when settled on the planar surface, a detector configured to generate a signal indicative of photons emitted from the illuminated detection molecules, and a processor configured to determine presence of the detection molecules based at least in part on the signal from the detector.

The present invention relates to a so-called image-based assay, or planar assay, in which the fluorescent signals from the detection molecules are acquired when the particles are arranged on a planar surface. The particles may be randomly arranged on the planar surface and their position determined by means of an additional fluorescent signal emitted from one or more dyes of the particles. This additional signal may also be used to determine identity or type of the particles, as will be described in the following with reference to some embodiments of the invention.

The present invention offers advantages compared to flow-based assays. Firstly, image-based assays may be more accessible and easier to use compared to flow-based assays, which typically require more trained operators for handling and maintenance. Arranging the sample on a planar surface, such as on a multi-well plate, typically makes the handling easier compared to techniques wherein the sample is provided in a flow that passes through a flow cell. Secondly, the present invention allows for a no-contact readout of the sample, inherently protecting the instrument from contaminating pathogens and nullifying clogging issues. Thirdly, arranging the sample on the planar surface often makes it possible to store the sample and re-analyse it later. This is generally not the case in flow-based assays, where the sample is usually analysed immediately.

Further, the inventor has discovered a previously unrecognised problem of aggregation of the particles on the planar surface. It is believed that the aggregation may be caused by magnetic interaction between the particles, possibly due to remaining magnetisation of the particles. It has been observed that at least some of the particles may aggregate, i.e., group together, agglomerate, cluster, or form larger structures, on the planar surface both as they settle on the surface and over time. This issue has also been observed for particles which, according to the particle provider, are said to exhibit superparamagnetic properties. The aggregation may interfere with the analysis of the sample, as it may be difficult to discriminate between neighbouring particles and their respective fluorescent signal. Further, the aggregation may complicate the determination of the particle centres, which is a key parameter in image-based analysis using multiple dyed particles when determining a colocalised analyte signal in the fluorescent images specific for the illuminated detection molecules.

The inventor has realised that the aggregation may be reduced if the particles are exposed to an alternating magnetic field before they settle on the planar surface. Exposing the particles to the alternating magnetic field may be referred to as a "demagnetisation" or "degaussing" of the particles, which may be understood as a reduction or a substantially complete elimination of magnetisation of the particles.

The particles may be exposed to the alternating magnetic field in connection with an agitation of the sample. The agitation is typically performed to reduce any aggregation and form a suspension in which the particles are substantially evenly distributed in a liquid medium. The particles may be exposed to the magnetic field prior to the agitation, during the agitation, or after the agitation. In some examples, the particles may be at least partly agitated by the exposure to the magnetic field.

After the exposure to the magnetic field, the particles may be allowed to settle on the planar surface. This may be achieved by allowing the sample to rest, while relying on the influence of the gravitational force to cause the particles to sink towards the planar surface. It is however possible to assist the settling by applying external forces, such as centrifugal forces generated by a centrifuge.

The magnetic field may be generated by one or more electromagnets, which may be operated to alternate or vary the direction of the magnetic field to which the particles are exposed. It may also be possible to generate the magnetic field by one or more permanent magnets, which may be moved to alternate or vary the field direction.

In some embodiments, each of the plurality of particles may comprise a magnetic material, such as a ferromagnetic material. The ferromagnetic material may, for instance, be provided in the form of magnetic particles interspersed in the material forming the particle. Beneficially, the magnetic material may increase the ease and speed of separation. By applying a magnetic field, the particles (along with any bound analyte) can be more rapidly pulled out of solution and separated from unbound components at a reduced sample loss. This may simplify the washing steps of the assay and reduce the time required for these steps compared to traditional centrifugation or filtration methods.

In some examples, the particles may comprise a predominantly superparamagnetic material. In different words, the particles may comprise magnetic particles of a size that only allows a single magnetic domain to be formed in the particle. As is well known in the art, superparamagnetic materials typically exhibit a relatively high magnetic susceptibility, allowing them to magnetise relatively strongly in an external field. Thus, the particles may be controlled relatively efficiently during, for example, washing and separation. Further, superparamagnetic materials are also known to exhibit substantially zero remanent magnetisation when the external magnetic field is removed. Beneficially, this may reduce their tendency to form clusters on the planar surface.

However, by applying the alternating magnetic field as described above, the aggregation may be further decreased. This may be caused by the alternating magnetic field removing or at least reducing a remaining magnetisation in the particles, also for predominantly superparamagnetic particles. The alternating magnetic field may help to reduce the alignment of the magnetic moments of the magnetic domains of the particles, thereby increasing the degree of randomisation of the domains.

The particles may have a shape conforming to a sphere, and may also be referred to as microspheres, beads, or microbeads. Other shapes and forms are however possible, and it is appreciated that the inventive concept as described herein may not necessarily be limited to spherical particles. Other suitable shapes include rods, stars, tubes, plates, wires, and shells (ball-shaped particles with a hollow core). However, for the purpose of the present disclosure, the terms "particles" and "microspheres" are used interchangeably to illustrate some examples of suitable particles.

The detection molecules may comprise or be conjugated to a fluorescent label, also known as a fluorophore or fluorescent dye, which is configured to absorb light at a certain wavelength and typically re-emit light at a longer wavelength. Different fluorescent labels may be used to distinguish between different detection molecules that bind to different analytes. Each fluorescent label may have a unique emission spectrum, allowing them to emit light at different wavelengths when excited by a light source. The use of different fluorescent labels allows for a simultaneous detection of multiple analytes in a single sample, as each analyte can be associate with a unique fluorescent signal. Examples of fluorescent labels range from small organic dyes to large quantum dots and nanoparticles.

The detection molecules may include antibodies, which may be understood as proteins that are able to bind to specific target analytes, such as antigens, with high specificity and affinity. Further examples include aptamers, which are understood as short strands of DNA or RNA engineered to fold into specific three-dimensional shapes that can bind to specific target molecules. Yet further examples include peptides and various enzymes.

In some examples, the detection molecules may be configured to bind to the analyte, which in turn may bind to the particle via a capture molecule. The capture molecule may be configured to selectively bind to and isolate specific analytes from a sample. The capture molecule is typically bound to a solid support, such as a particle, to allow for easy separation of the analyte from the rest of the sample. Typical examples of capture molecules include antibodies, aptamers, peptides, and lectins.

In some examples, each particle may be dyed with a fluorophore, which may be similarly configured as the fluorophores discussed above for the detection molecules. The light emitted from the dye may then be used to determine the position of the particle on the planar surface. The position, which may be represented by a centre of the particle, may hence be determined without any using any dedicated structures on the planar surface, such as grooves or indentations, commonly used for positioning the particles in prior art technologies. Information about the position of the particle may be used to increase efficiency and precision of the analysis of the emitted light signal from the fluorophore-labelled detection molecules. The determined position may, for example, be used to delimit the region of interest in an image of the planar surface, in which the signal can be expected to be found. The position of the particles may also make it easier to identify and distinguish signals from two or more particles that are arranged relatively close to each other.

The particles may also be dyed to enable multiplexing. In some examples, the particles are dyed with different fluorescent dyes. As already mentioned, each dye may have a unique emission spectrum which allows the dye to be distinguished from others when excited with light of a specific wavelength. By using different combinations of dyes, a relatively large number of distinctly coloured particle subsets can be created. By allowing each subset to be capable of capturing a specific analyte, the multiplexing capacity of the assay may be increased.

In an example, each subset of particles may be dyed with one or more fluorescent dyes. The concentration of each dye used may be varied to create particles with different colour intensities. By coupling each uniquely coloured subset of particles with a capture molecule that is specific to a particular analyte, the dyed subsets may be mixed, and the different colours used to distinguish between the subsets and hence the different analytes. Reducing the degree of aggregation of the particles may be particularly beneficial in connection with multiplexing, as this may make it easier to discriminate between different subsets of particles and their colocalised analyte signals.

Hence, in an example, a first subset of the particles may be dyed with a first concentration of a first type of fluorophore and a second concentration of a second type of fluorophore. Further, a second subset of particles may be dyed with a third concentration of the first type of fluorophore and a fourth concentration of the second type of fluorophore. A ratio between the first and the second concentrations may be larger than a ratio between the third and fourth concentrations. It may then be possible, for each one of the plurality of particles, to determine that the particle forms part of the first subset or the second subset based at least in part on a difference in intensity of the detected photons emitted from the first type of fluorophore and the second type of fluorophore, respectively.

The inventive concept may be applied to any investigative technology involving the analysis of a plurality of particles, such as microspheres or microbeads, arranged on a planar surface. The confocal scanning microscopy discussed in the following is merely to be understood as an illustrative example.

Further features and advantages of the invention will become apparent from the following description of preferred embodiments of the invention, given by way of example only, which is made with reference to the accompanying drawings.

### Brief Description of the Drawings

Figure 1 shows schematically a system according to an embodiment of the present invention.
Figure 2 shows schematically fluorophore-labelled detection molecules bound to a particle according to an embodiment of the present invention.
Figure 3 are photos of particles when settled on a planar surface.
Figure 4 is a flow chart illustrating a method according to an embodiment of the present invention.

### Detailed Description

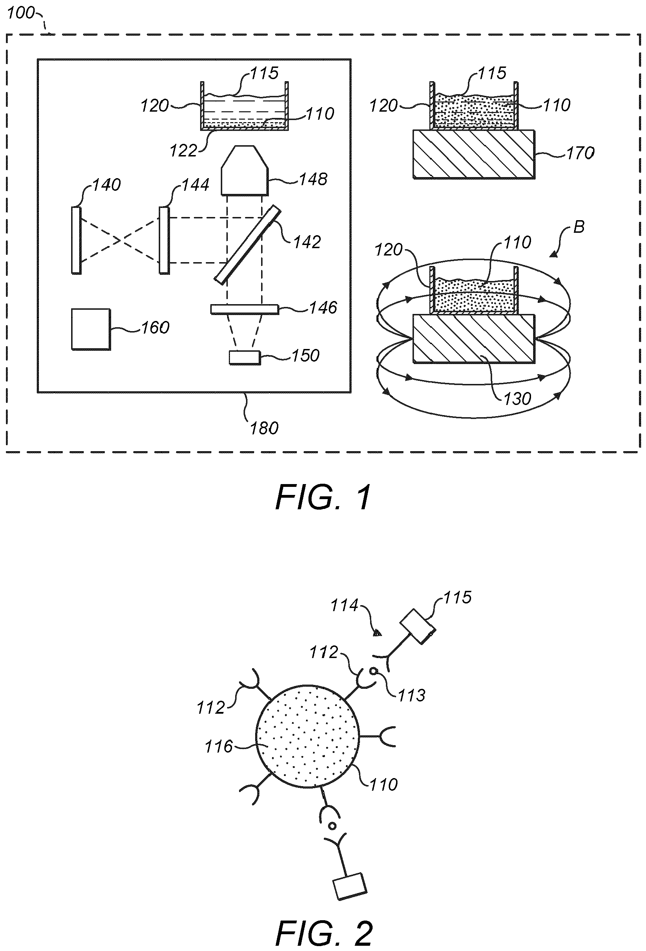

Figure 1 schematically illustrates a system 100 for determining presence of a plurality of fluorophore-labelled detection molecules bound to a plurality of particles 110. The system 100 comprises an illumination means 140 configured to illuminate the particles 110 when settled on a planar surface 122 of a sample holder 120, and a detector 150 configured to generate a signal indicative of photon emitted from the fluorophore-labelled detection molecules when illuminated. Further, a processor 160 is provided to determine presence of the detection molecules based on the signal from the detector 150. The illumination means 140, the detector 150, and the processor 160 may form part of a reader 180, such as a confocal laser scanning microscope 180. The inventive concept, in which aggregation of the particles 110 may be reduced by exposure to an alternating magnetic field, may however be employed in other types of assays as well, using other types of imaging techniques than confocal laser scanning microscopy.

The system 100 may also comprise an agitator 170 configured to agitate the sample, and a magnetic field generator 130 configured to expose the sample to an alternating magnetic field B.

It is to be noted that the various components of the above system 100, i.e., the illumination means 140, the detector 150, the processor 160, the agitator 170, and the magnetic field generator 130, may be provided as structurally separate components or integrated in one or more common platforms. Thus, in an example, the illumination means 140, the detector 150, and the processor 160 may be provided in the reader 180, whereas the agitator 170 and the magnetic field generator 130 may be provided as separate, freestanding units. In some examples, the magnetic field generator 130 and the agitator 170 may be integrated into a single unit. In further examples, the processor 160, or at least parts of its functionality, may be arranged at a separate location, such as in a separate server forming part of a local area network or in a cloud-based server.

The particles 110 may be provided in a liquid medium 115, such as distilled water or buffer solution such as phosphate-buffered saline (PBS), accommodated in a container 120. The container 120 may, for example, be a well of a multi-well plate (not shown). Figure 1 shows the sample in three different situations - when being exposed to an alternating magnetic field B from the magnetic field generator 130, when being agitated by the agitator 170, and when being analysed in the reader 180. The agitator 170 may be employed to help distribute the particles 110 throughout the medium 115, thereby creating a particle suspension. The particles 110 may then be allowed to settle on the planar surface 122 forming the bottom of the container 120, before the sample is analysed in the reader 180. It will be appreciated that the particles 110 may be exposed to the alternating magnetic field B when suspended in the liquid medium 115 (as illustrated in figure 1), when settled on the planar surface 122, or when arranged in any state therebetween.

The illumination means 140 and the detector 160 may be configured to illuminate the sample and detect photons emitted or scattered from the sample. The light from the illumination means, or light source 140, may be directed to the sample by a first optical system, for example comprising a lens 144, a beam splitter 142, and an objective lens 148. The first optical system may be configured to direct the light in a scanning motion over the sample, causing fluorescent labels of detection molecules or dyes of the particles 110 to emit fluorescent light. The sample may hence be scanned by at least two different types of light - a first light having a wavelength exciting the fluorescent labels of the detection molecules, and a second light having a wavelength exciting the dye(s) of the particles 110. In an example, light with a wavelength of 532 nm may be used for generating a signal indicative of the analyte, whereas 638 nm may be used for generating a signal indicative of the particles 110.

The scanning may, for example, be achieved galvanometer mirrors that are rapidly moved to scan the light across the sample. The fluorescent light may be collected by the objective lens 148, collimated, and passed through the beam splitter 142. A second optical system 146 may direct the fluorescent light to the detector 150, which preferably may be a photodetector configured to generate a signal that can be processed by the processor 160. Further, a sample stage (not shown) may be provided, which is configured to move the sample in various directions to allow for scanning and focusing on different parts on the sample.

However, it is to be understood that the specific components and the exact setup of the system 100 disclosed herein are exemplary only. For example, the reader 180 may very well use multiple light sources 140 emitting light with different properties, such as wavelengths, and/or optical filters for providing a desired colour of the light and enable multiplexing. Furthermore, the configuration of optical systems, including for instance mirrors, beam splitters, apertures, pin holes, and filters may vary.

As shown in figure 1, the sample may be analysed from below, through the bottom of the container. Accordingly, the planar surface 122, onto which the particles 110 may rest when settled, may be formed of a light-transmitting material that allow light to pass from the illumination means 140 to the sample and fluorescent light generated by the sample to escape through the planar surface 122 to the detector 150. The bottom of the container may, in some examples, be formed of a light-transmitting or transparent plastic material or glass. In alternative examples, however, the sample may be illuminated from above, through the liquid medium. The detector 150 may be arranged below the sample or above the sample.

The agitator 170 may be employed to create motion of the particles 110 in the liquid medium 115, typically by rotation or vibrations, to keep the particles 110 substantially uniformly distributed in the liquid medium 115 and reduce the degree of aggregation. The vibrations may be imparted to the sample by placing the container 120, such as the multi-well plate, in mechanical contact with a rotating part of the agitator 170.

The magnetic field generator 130 be an electrically operated device comprising one or more electromagnetic coils, which may be combined with a capacitor to create one or more oscillating LC circuits. When connected to a power source, the LC circuit starts generating an oscillating current which, in turn, creates an oscillating magnetic field B. The particles 110 may be placed in the magnetic field B, for example by placing the container 120 on a dedicated surface of the magnetic field generator 130, to increase the randomisation of the magnetic domains of the magnetic material of the particles 110. The sample may be moved or rotated to allow the magnetic field B to pass through the sample from different directions or angles. According to some observations, the magnetic field B may reduce or neutralise the magnetic field in each particle 110 by changing the alignment of the magnetic domains within a magnetisable material of the particles 110. In a magnetised particle, the magnetic domains are typically aligned in the same direction, which creates the particle's overall magnetic field. However, when exposed to the alternating field B generated by the magnetic field generator 130, the magnetic domains may begin to change direction and follow the pattern of the external field. By designing the magnetic field generator 130 to repeatedly change direction of the generated magnetic field B, the magnetic domains in the particles 110 may be caused to flip back and forth. As the strength of the magnetic field decreases, for example by increasing the distance between the magnetic field generator 130 and the particles 110, these flips may become less complete, eventually leaving a major part of the domains in random directions. The randomisation of the magnetic domains means that their individual magnetic fields largely cancel each other out, leading to the particle having little to no overall magnetisation. Preferably, the particles 110 may be slowly removed from the magnetic field B while the field is still active to hinder the magnetic domains from re-aligning themselves to any residual field. The power, frequency, and duration of the demagnetising process can depend on factors like the type of magnetic material of the particles 110, the size and shape of the particles 110, and the strength and orientation of any initial magnetisation of the particles 110. The same applies to the direction(s) or orientation of the alternating field, which may vary depending on the type of material and any initial or remaining magnetisation of the particles 110. Thus, it will be appreciated that the magnetic field B may be caused to alternate in one, two or three dimensions

In an exemplary sequence, the container 120 comprising the sample is placed on the magnetic field generator 130 to expose the sample to the alternating magnetic field B, then moved to the agitator 170 where the sample is agitated to distribute the particles 110 more evenly in the fluid medium 115. By moving the container 120 tot the agitator 170, the sample may experience a gradual reduction of the field strength as the sample moves away from the magnetic field generator 130. The sample may then be set aside to rest until at least some of the particles 110 have settled on the planar surface 122 of the bottom of the container 120. This process may typically take 10-20 minutes, depending on the type of particles and the type of liquid medium. Thereafter, the sample may be inserted into a sample stage of the reader 180. In case the samples are arranged in a multi-well plate, the sample stage may be operated to move each well into the field of view of the of the detector 150 and the reading of the fluorescent signals be initiated by scanning the sample with light from the illumination means 140. The imaging of the samples may be substantially automatic, requiring no or very little intervention from the operator.

Figure 2 is a cross section of a particle 110, to which a plurality of fluorophore-labelled detection molecules 114 are bound. The particle 110 and detection molecules 114 may be similarly configured as the ones discussed above in connection with figure 1. In the present example, the detection molecules 114 are bound to the particle 110 via an analyte 113, which in turn is bound to a detection molecule 112 attached to the surface of the particle 110. Each of these components will be discussed in greater detail in the following. It is to be noted that figure 2 is a schematic representation of the functional elements of the particle 110 and the associated molecules/substances and that dimensions and measures may be exaggerated for illustrative purposes.

The particle 110 may be a magnetic microsphere 110, also referred to as a bead or microbead. The microsphere 110 provides a solid support for the capture molecules 112, which are bound to the surface of the microsphere 110. The microsphere 110 may typically be formed of a polymer matrix with magnetic particles 116 dispersed therein. The polymer matrix may provide structural stability to the microsphere 110 and a surface that can be modified for the attachment of capture molecules 112, while the magnetic particles 116 may provide the microsphere 110 with its magnetic properties. Various types of polymers can be used for the matrix, including polystyrene and poly(lactic-co-glycolic acid)(PLGA), as will be known to a person with ordinary skill in the art.

The particles 110 can be utilised in a variety of shapes and sizes, typically ranging from nanometres to micrometres in size, depending on the particular requirements of the assay, such as signal intensity and the need for specific surface chemistries. Relatively small microspheres 110 may have a beneficial surface-to-volume ratio, which for a given volume provide a relatively large surface onto which the capture molecules 112 may be attached. In absolute numbers, however, a larger microsphere 110 has a larger surface to which more capture molecules 112 can be bound. This, in turn, allows for more analyte 113 and detection molecules 114 to be bound to each particle 110, resulting in an increased intensity of the fluorescence signal emitted from the particle 110.

Larger particles are also easier to capture in a magnetic field and are therefore less easily lost during, for example, washing and separation. A drawback, however, is that the larger particles have been observed to aggregate more easily. By exposing the particles 110 to the alternating magnetic field, the size of the particles 110 may be increased without at the same time increasing their tendency to aggregate. In some examples, the microspheres 110 have an average diameter that lies in the range of 2-10 µm, such as 4-8 µm. In the present example, the microsphere 110 has an average diameter of about 8 µm.

The magnetic particles 116 may be formed of a ferromagnetic material, such as magnetite (Fe₂O₄) or maghemite (γ-Fe₂O₃) and may have a size that allows the particles 116 to exhibit predominantly superparamagnetic properties.

In some examples, the particle 110 may be coated with a layer protecting the magnetic particles 116 from exposure to the surrounding environment. The coating may further facilitate the attachment of capture molecules 112.

Further, the microsphere 110 may be dyed with one or more types of fluorophores, or dyes (not shown) to enable identification of the microsphere 110 on the planar surface. Two or more subsets, or populations, of microspheres can be dyed with two or more types of dyes, each having a different emission spectrum. By varying the relative concentration of the dyes between different populations, the populations may be identified based on the intensity composition of the fluorescence emitted by the dyes.

The capture molecules 112 are provided to selectively bind the target analyte 113 and immobilise the analyte 113 on the solid support provided by the microsphere 110. In an example, the capture molecules 112 are antibodies, such as monoclonal antibodies, that are specific to a particular epitope. Hence, the capture molecules 112 may also be referred to as capture antibodies 112.

The analyte 113 is the specific substance or molecular target that the assay is designed to detect. The nature of the analyte 113 may vary widely, depending on the purpose of the assay. In an example, the analyte 113 is a protein, such as a protein found in blood. The protein may, for example, be a prostate-specific antigen or PSA, specific antibodies (such as those produces in response to a viral infection like HIV) or signalling proteins like cytokines (such as Interleukin-6 or IL-6 in inflammatory responses).

The detection molecules 114 are typically designed to bind to a portion of the analyte 113 which is different from the portion to which the capture molecules 112 bind. The detection molecules 114 and may be labelled with a fluorophore 115 which emits light of a specific wavelength when excited and may in some examples be referred to as fluorophore-conjugated antibodies. By providing different detection molecules 114 with different fluorophores 115, each having a unique excitation an emission spectrum, simultaneous detection of multiple analytes in a single sample may be allowed. This may also be referred to as multiplexing.

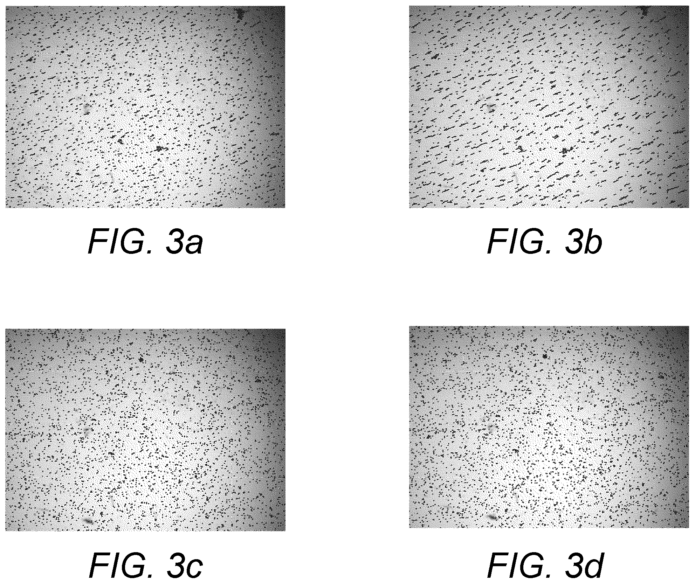

Figures 3a-d are photographs of microspheres 110 arranged on a planar surface as discussed above in connection with figure 1. The photographs originate from an experiment in which two identical samples comprising microspheres 110 suspended in a liquid medium were agitated, using an agitator as discussed in connection with figure 1. The particles were then allowed to settle on the planar surface. The particles 110 of the first sample are shown in figures 3a and b, with figure 3a showing the particles 10 minutes after the agitation and figure 3b showing the particles 40 minutes after the agitation.

The particles 110 of the second sample are shown in figures 3c and d, with figure 3c showing the particles 10 minutes after the agitation and figure 3d showing the particles 40 minutes after the agitation. The second sample however differs from the first sample in that the particles 110 was exposed to an alternating magnetic field, using a magnetic field generator as discussed in connection with figure 1, prior to the agitation. An objective of the experiment was thus to investigate if the exposure to the alternating magnetic field has any effect on the degree of aggregation, both in the short term (10 minutes) and in the long term (40 minutes). In this experimental setup, 30 minutes is the time it takes for the reader to scan an entire multi-well plate. The photographs taken after 10 minutes can therefore be considered to represent the first well that is scanned, and the photographs taken after 40 to represent the last well to be scanned.

Comparing figure 3a with figure 3b, showing the sample that was not exposed to the alternating magnetic field, it can be concluded that the initial degree of aggregation increases over time. There are quite a few, relatively small clusters formed after 10 minutes, which after 40 minutes have merged into larger clusters. This trend, with clusters forming and merging over time, is not visible in figures 3c and d, showing the sample that has been exposed to the alternating magnetic field. In addition, when comparing figure 3a (10 minutes, not demagnetised) with figure 3c (10 minutes, demagnetised), the initial degree of aggregation is significantly lower for the sample in figure 3 c.

In conclusion, the degree of aggregation can be significantly reduced by exposing the particles to an alternating magnetic field before they are allowed to settle on the planar surface. This effect seems to last at least over the 40 minutes during which the particles were observed in the experiment and has been observed to continue for several hours afterwards, as long as the particles are kept in solution.

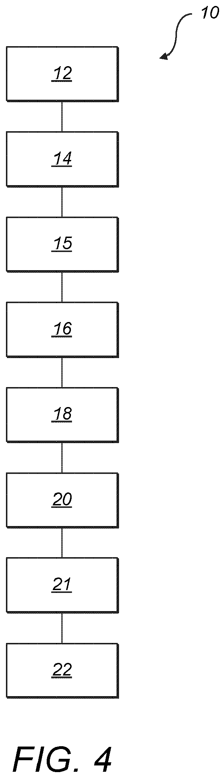

Figure 4 is a flow chart outlining a method 10 for determining presence of a plurality detection molecules according to some embodiments of the present invention. The detection molecules may be bound to a particle, such as a magnetic microsphere as discussed above with reference to figure 2, using a system which may be similarly configured as the one shown in figure 1.

According to the example shown in figure 4, the method 10 comprises providing 12 a sample comprising the particles suspended in a liquid medium. It is to be noted that the particles may have undergone some processing in previous steps, which are not disclosed in figure 4. In previous steps, the particles may, for example, be dyed with one or more fluorescent dyes to allow detection and identification of the particles, as well as be functionalised, i.e., treated to introduce reactive groups on their surface. These groups may be chemically reactive and allow for a covalent attachment to capture molecules to be formed. The functionalised particles may be mixed with capture molecules to allow the capture molecules to become covalently attached to the reactive groups on the surface of the particles. After this process, excess capture molecules may be washed away, leaving behind particle with capture molecules bound to their surface. The capture molecules may then be exposed to the target analyte, which binds to the capture molecules, whereas unbound substances may be washed away after a certain incubation period. Finally, the particles may be exposed to the fluorophore-labelled detection molecules, which may bind to the analyte, whereafter excess detection molecules may be washed away.

The resulting particles may be complexes of particle-capture molecule-analyte-detection molecules provided in step 12. In the next step, the particles may be exposed to an alternating magnetic field to reduce a degree of aggregation of the particles. Additionally, the particles may be agitated 15 to further reduce the degree of aggregation. Thereafter, the particles may be allowed to settle 16 on a planar surface.

When at least some of the particles, such as a major part of the particles, have settled on the planar surface, the particles may be illuminated 18 by a light source, such as a laser of a confocal laser scanning microscope. The resulting fluorescent light emitted from the fluorescent label of the detection molecules may then be detected 20, and the presence of the detection molecules determined 22 based at least in part on the detected photons. In some examples, the method 10 may further comprise a step of determining 21 that the particles form part of a specific subset or population of particles, based at least in part on a difference in intensity of the detected photons emitted from a first type of dye and a second type of dye of the particle.

The above embodiments are to be understood as illustrative examples of the invention. Further embodiments of the invention are envisaged. For example, the particles may not necessarily be limited to microspheres. Other particles are also envisioned, such as rods or ellipsoids. In addition, other types of imaging techniques may also be possible, including fluorescence microscopy. It is to be understood that any feature described in relation to any one embodiment may be used alone, or in combination with other features described, and may also be used in combination with one or more features of any other of the embodiments, or any combination of any other of the embodiments. Furthermore, equivalents and modifications not described above may also be employed without departing from the scope of the invention, which is defined in the accompanying claims.

## Claims

1. A method for determining presence of a plurality of fluorophore-labelled detection molecules (114) bound to a plurality of particles (110) arranged on a planar surface (120), comprising:
providing (12) a sample comprising the particles suspended in a liquid medium (115);
exposing (14) the particles to an alternating magnetic field (B) to reduce a tendency of the particles to aggregate on the planar surface;
allowing (16) the particles to settle on the planar surface;
illuminating (18) the particles;
detecting (20) photons emitted from the illuminated detection molecules; and
determining (22) the presence of the detection molecules based at least in part on the detected photons.

2. The method according to claim 1, wherein each detection molecule is bound to an analyte (113), wherein the analyte is bound to a capture molecule (112), and wherein the capture molecule is bound to the particle.

3. The method according to claim 2, wherein the capture molecule is a capture antibody, and wherein the analyte is a protein found in blood.

4. The method according to any of the preceding claims, wherein each particle is dyed with a fluorophore, and wherein the method further comprises detecting photons emitted from the fluorophore to determine the position of the particle on the planar surface.

5. The method according to claim 4, wherein:
a first subset of the particles is dyed with a first concentration of a first type of fluorophore and a second concentration of a second type of fluorophore;
a second subset of the particles is dyed with a third concentration of the first type of fluorophore and a fourth concentration of the second type of fluorophore; and
a ratio between the first and second concentrations is larger than a ratio between the third and fourth concentrations.

6. The method according to claim 5, further comprising, for each of the plurality of particles, determining (21) that the particle forms part of the first subset or second subset based at least in part on a difference in intensity of the detected photons emitted from the first type of fluorophore and the second type of fluorophore, respectively.

7. The method according to any of the preceding claims, wherein each of the plurality of particles comprises a ferromagnetic material, and wherein the exposing the ferromagnetic material to the alternating magnetic field comprises at least partly randomizing magnetic domains of the ferromagnetic material.

8. The method according to claim 7, wherein the ferromagnetic material is provided as a plurality of magnetic particles (116) having a size allowing them to be predominantly superparamagnetic.

9. The method according to any of the preceding claims, wherein each of the plurality of particles is substantially spherical, and wherein an average diameter of the plurality of particles lies in the range of 2-10 µm, such as 4-8 µm.

10. The method according to any of the preceding claims, further comprising agitating (15) the sample to further reduce the tendency of the particles to aggregate.

11. The method according to any of the preceding claims, wherein the exposing the particles to an alternating magnetic field comprises gradually reducing a strength of the magnetic field.

12. A system (100) for determining presence of a plurality of fluorophore-labelled detection molecules (114) bound to a plurality of particles (110) arranged on a planar surface (122), comprising:
a magnetic field generator (130) configured to expose a sample, comprising the particles (110) suspended in a liquid medium (115), to an alternating magnetic field (B) to reduce a tendency of the particles to aggregate on the planar surface;
an illumination means (140) for illuminating the particles when settled on the planar surface;
a detector (150) configured to generate a signal indicative of photons emitted from the illuminated detection molecules; and
a processor (160) configured to determine presence of the detection molecules based at least in part on the signal from the detector.

13. The system according to claim 12, further comprising an agitator (170) configured to agitate the sample to further reduce the tendency of the particles to aggregate, wherein the agitator is structurally integrated with the magnetic field generator.

14. The system according to claim 12 or 13, wherein each particle is dyed with a fluorophore, wherein the detector is configured to generate the signal indicative of photons emitted from the fluorophore, and wherein the processor is configured to determine the position of the particles based at least in part on the signal from the detector.

15. The system according to any of claims 12 to 14, wherein the illumination means and the detector form part of a confocal laser scanning microscope.
